# EUROPEAN PATENT SPECIFICATION

(11) **EP 2 874 682 B1**
(45) Date of publication and mention of the grant of the patent: **05.07.2017**
(21) Application number: 13734400.8
(22) Date of filing: 05.07.2013
(51) Int. Cl.: A61M 5/145, G06F 1/16, A61M 5/14, A61M 5/142, A61M 5/31

(54) **SYRINGE PUMP WITH PIVOTABLE DISPLAY**
SPRITZENPUMPE MIT SCHWENKBAREM BILDSCHIRM
POMPE À SERINGUE AVEC ÉCRAN PIVOTANT

(30) Priority: 20.07.2012 EP 12177303; 12.09.2012 US 201261699930 P
(43) Date of publication of application: 27.05.2015
(73) Proprietor: Fresenius Vial SAS, 38590 Brézins (FR)
(72) Inventor: WOLFF, Rémy, F-38210 Morette (FR); PONCON, Gilbert, F-38340 Pommers la Placette (FR)
(74) Representative: Kusche, Robert
(86) International application number: PCT/EP2013/064243
(87) International publication number: WO 2014/012802

(56) References cited:
- EP-A2- 1 074 221
- EP-A2- 1 374 932
- WO-A1-90/07742
- US-A- 5 335 142
- US-A1- 2005 230 575
- US-A1- 2006 079 768
- US-A1- 2011 261 515

## Description

The invention concerns a medical pump comprising a pumping mechanism located in a housing and a display placed on a support member which is articulated on the housing, the support being pivotable with respect to the housing around a first axis between a folded-back position and an folded-out position.

A syringe pump comprising a housing including a space which is configured to accommodate a syringe to be placed within the housing and is closable by a cover provided with a window, the cover including an operation panel and a display means is described in document EP 1 374 932 A1.

The drawback of this pivotable cover is that the display and the keyboard are directed downwards when the cover is in the open position. If the pump is situated under the level of the user's eyes, the user cannot read the information displayed or enter data with the help of the keyboard, unless he knees, what is not a convenient working position.

Document WO 2009/135667 A1 is concerned with an infusion pump for dispensing medication comprising a pull-out display unit that can be exposed to a user.

An intravenous fluid infusing device having a built-in display and comprising a foldable casing with pumping apparatus mounted therein is known from document EP 0 399 119 A1. A folding display is hingedly connected to the casing and is movable between an open position for displaying instructions and information to the user to allow operation of the device, and a closed position where the display is protected from view. This display is not visible when the door is open, i.e. when the pump is stopped. It is therefore not possible to use the display for observing the progress of the perfusion.

Furthermore, it is current to put several pumps one above the other by fixing them to a support. In this case, the available place between the lower side of a pump and the upper side of the pump located directly under this pump is limited. If the door is quite large, as it is common for instance in the field of peristaltic pumps, the lower front angle of the door may come into contact with the upper side of the pump situated directly under this pump well before the door is fully opened.

This may damage the pump at the contact point and restrict the access to the pump, especially for changing the tubing (peristaltic pumps) or changing the syringe (syringe pumps).

The object of the invention is to facilitate the handling of a display of a medical pump so that the user can see the display irrespective of the support member being in the folded-back or in the folded-out position without changing his position. Another object of the invention is to allow a complete opening of the door of the pump even if the space underneath the lower side of the door is restricted.

This object is achieved according to the invention in that the display is pivotable with respect to the support member around a second axis between a first position, called non pivoted position, and a second position, called pivoted position, wherein the first axis and the second axis are parallel to each other.

This measure allows in a first movement to make pivot the support member around the first axis and in a second movement making pivot the display around the second axis. These two movements are independent from each other: It is therefore possible to make them simultaneously or subsequently according to the requirements. Another advantage lies in the fact that it is possible to share the opening movement of the door over two axes: the first one, situated behind, close to the housing of the pump, and the second one, situated in front with more distance to the housing. The first axis which is close to the housing allows an almost horizontal approach of the door at the end of the closing movement (or at the beginning of the opening movement) whereas in its further opening movement, the door pivots around the second axis, placed in front, which allows a larger opening of the door without coming into contact with the pump situated underneath or the support onto which it is placed. This is especially of interest if the frame holding the display is particularly thick.

According to the invention, the display may be placed in a frame which is pivotable with respect to the support member in order to pivot around the second axis between the non pivoted position and the pivoted position.

In this case, it is possible that the frame is provided with a keyboard which is preferably placed aside the display.

According to the requirements, the support member may be configured to rotate around the first axis from the folded-back position to the folded-out position in a direction which is opposed or identical to the direction of rotation of the display or the frame of the display from the non pivoted position to the pivoted position.

Preferably, the support member and the display or the frame holding the display block the access to the pumping mechanism when the support member is in the folded-back position and the display or the frame is in the non pivoted position, whereas when the support member is in the folded-out position, the pumping mechanism is accessible.

According to another aspect of the invention, the support member comprises an arbor and two arms parallel to each other and perpendicular to the arbor.

A further development of the invention consists of the fact that indexation means are provided for defining the privileged pivot positions of the support member with respect to the housing of the pump.

In this context, it is advantageous that the indexation means comprise on the one hand side a bushing fixed on the arbor and being provided with at least one depression and on the other hand side of a came provided with a projection, the came being placed in the housing of the pump and being mobile exclusively in translation parallel to the axis of the arbor and means being provided for pushing the projection of the came against the corner of the bushing provided with the depression(s).

According to a further embodiment of the invention, retaining means are provided with liberation means for preventing the display or the frame from pivoting in one direction with respect to the support member if the liberation means are not actuated.

In this context, it is favourable that the retaining means comprise on the one hand side a ratched wheel fixed on the support member and on the other hand side a pawl fixed on the display or the frame of the display.

The pump of the present invention may be a pump of the push-syringe type and the two pivot axes are preferably parallel to the axis of a syringe placed into the pump.

In a second embodiment of the invention, a spring is provided which is configured to keep the support member in the folded-out position if no external force is exerted on the support member. In this context, retaining means may be provided with liberation means for retaining the display or the frame holding the display in the closed position against the effect of the spring if the liberation means are not actuated. The pump is preferably a peristaltic pump and the two pivot axes may be parallel to the camshaft of the pumping mechanism. The display is placed preferably in a frame which is pivotable with respect to the support member in order to pivot around the second axis between the non pivoted position and the pivoted position and a counterplate my be placed on the part of the frame directed to the pumping mechanism when the support member is in folded-back position and the frame is in the non pivoted position.

In order to avoid a displacement of the tubing, the first axis may be placed in such a way that, when the display or the frame holding the display is in its non pivoted position and the support member (70) is moving to its folded-back position, the last part of the movement of counterplate is essentially horizontal and perpendicular to the axis. This may be achieved by placing the first axis essentially vertically below the place for a tubing located against the most protruding pumping finger of the pumping mechanism.

Furthermore, detecting means may be provided for detecting the position of the support member or of the display or the frame holding the display with respect to the housing of the pump.

An embodiment of the invention is hereafter described more in detail with reference to figures which show:
Figure 1: a pump according to a first embodiment of the invention with the frame holding the display in the closed position (support member in folded-back position and frame holding the display put against the support member in non pivoted position);
Figure 2: the pump of figure 1 with the frame holding the display in open position (support member in folded-out position and frame holding the display facing upwards in pivoted position);
Figure 3: a perspective view of the support member;
Figure 4: a perspective view of the rear side of the frame holding the display;
Figure 5: a perspective view of the support member and of the indexation means in folded-back position with the display (of which only the pawl and the fixation flange are shown) put against the support member in non pivoted position;
Figure 6: a perspective view of the support member and of the indexation means in folded-out position, the display (of which only the pawl and the fixation flange are shown) facing upwards in pivoted position;
Figure 7: a perspective view from the rear of the support member in folded-back position with the frame of the display put against the support member in non pivoted position;
Figure 8: a perspective view from the rear of the support member in folded-out position with the frame of the display facing upwards in the pivoted position;
Figure 9: a perspective view of a pump according to a second embodiment of the invention, with the frame holding the display in the closed position (support member in the folded-back position and frame in the non pivoted position);
Figure 10: the pump of figure 9 with the frame holding the display in the open position (support member in the folded-out position and frame in the pivoted position);
Figure 11: a perspective view of the support member of the pump of figure 9;
Figure 12: another perspective view of the support member of the pump of figure 9;
Figure 13: a perspective view of the support member in the folded-back position;
Figure 14: a perspective view of the support member in the folded-out position;
Figure 15: a perspective view of the frame holding the display in the closed position (support member in the folded-back position and frame in the non pivoted position);
Figure 16: a perspective view of the frame of the display in the open position (support member in the folded-out position and frame in the pivoted position);
Figure 17: a cross-section through the pump with the frame of the display in the closed position;
Figure 18: a cross-section through the pump with the frame of the display in the open position;
Figure 19 to
Figure 21: the opening movement of the frame of the display from the closed position to the open position via an intermediate position;
Figure 22: a schematic view (a) of the almost horizontal approach of the counterplate due to the pivoting movement around the first axis, (b) of the oblique approach of the counterplate due to the pivoting movement around the second axis and (c) of the closed position of the counter plate, wherein the tubing is adjacent to a slightly protruding pumping finger;
Figure 23: a schematic representation of the different pivoting directions.

The invention is presented with reference to two embodiments.

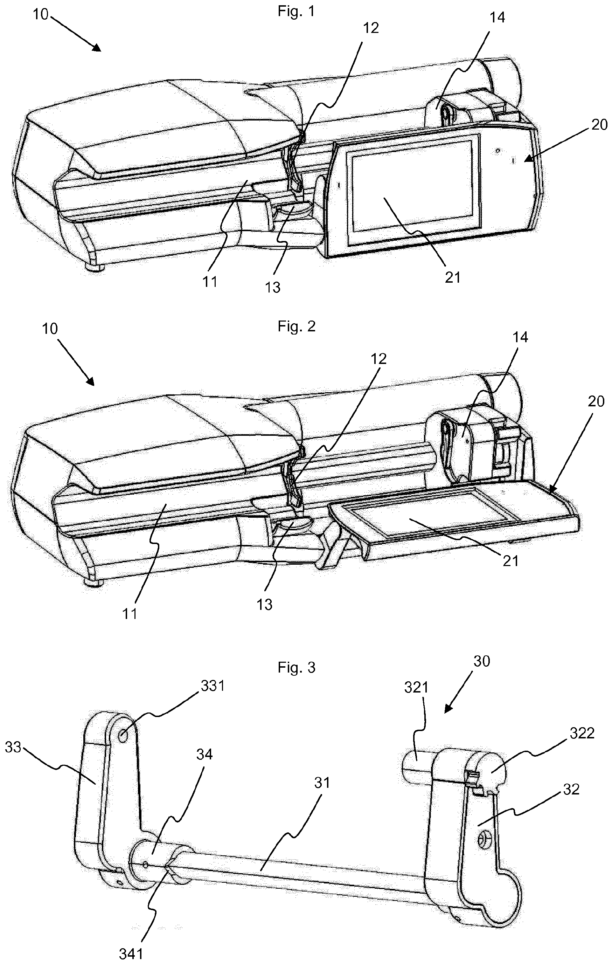

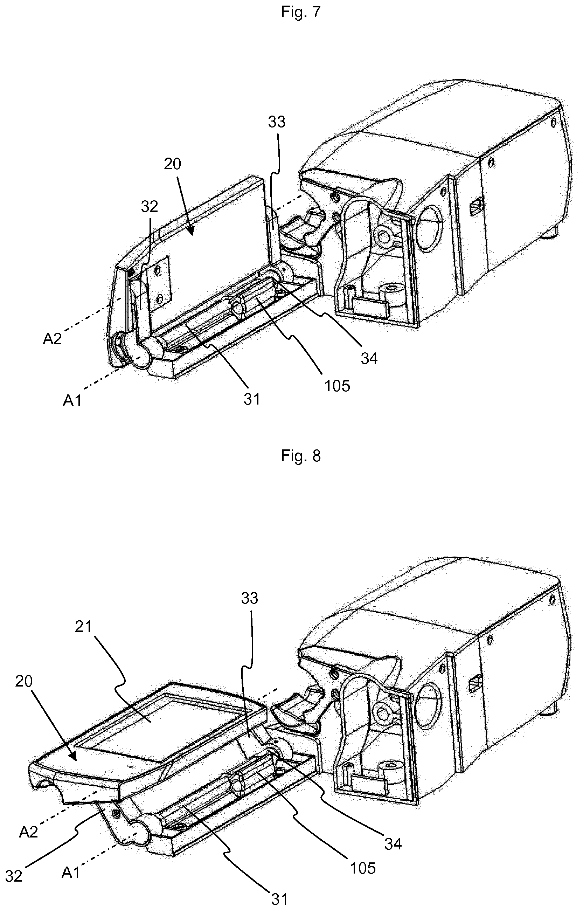

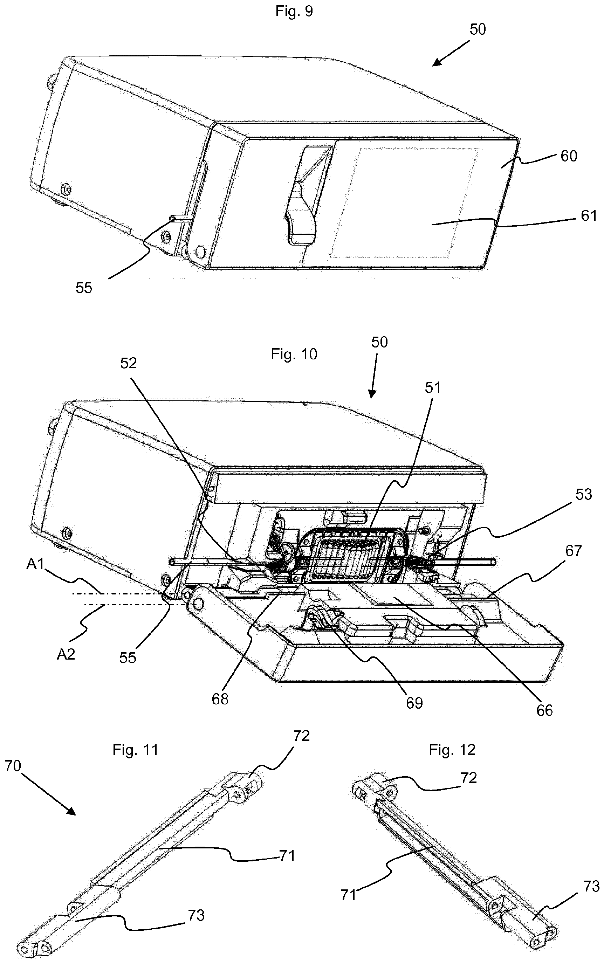

The pump shown in the figures 1 to 8 is a pump of the push-syringe type, whereas the pump shown in the figures 9 to 22 is a pump of the peristaltic type. It could also be any other apparatus equipped with a display which is able to pivot between a position in which it blocks the access to a pump, called closed position, and a discarded position in which the pump is accessible.

In the case of a push-syringe type pump, as shown in figures 1 to 8, the pump (10) is generally provided with a bed (11) for receiving a syringe and with means for blocking the syringe both in axial direction (12) and in radial direction (13). A pusher (14) can move in translation parallel to the axis of the syringe in order to push the piston thereof.

In the case of peristaltic pumps, as shown in figures 9 to 23, the pump (50) has a pumping zone in which are placed the fingers (51) and two holding members (52, 53) for holding in place a tubing on both sides of the pumping zone. In order to protect the pumping mechanism from external stresses, such as intrusion by foreign liquids or bodies, it is usual to place a flexible membrane (not shown) between the ends of the fingers and the flexible tubing.

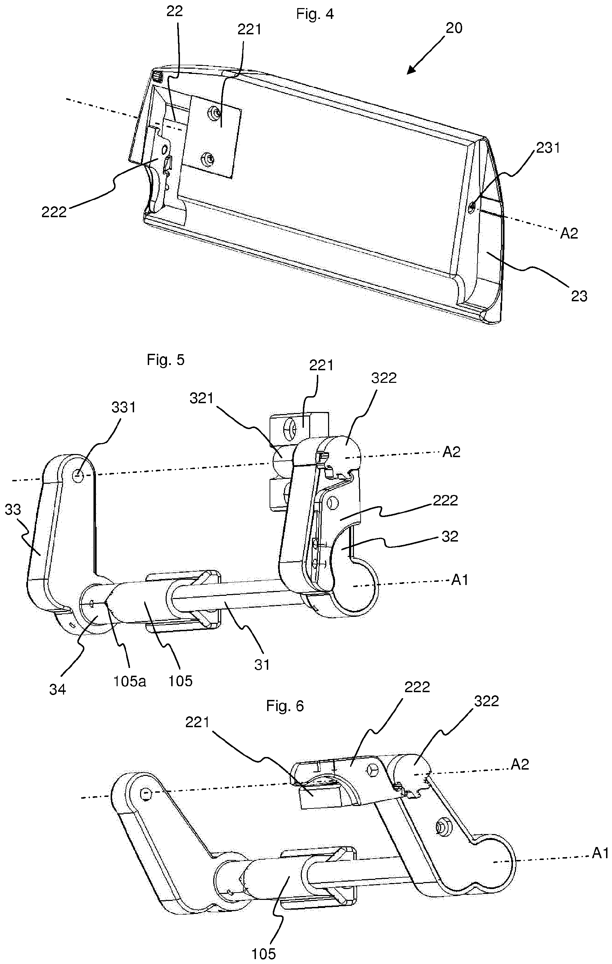

In both embodiments, a display (21, 61) is provided for displaying information concerning the functioning of the pump. This display (21, 61) is used both for programming the pump (10, 50) and for following the advancement of the pumping operation or for the indication of dysfunction or alert messages. The display (21, 61) is preferably placed in a frame (20, 60). The side of the frame which is opposed to the display can be seen in figures 4, 10 or 15. The display may be a tactile display. It may however be necessary to provide a keyboard in order to enter data into the control system of the pump. The keyboard can be placed beside the display.

The frame (20, 60) of the display is pivotingly fixed on a support member (30, 70) which itself is pivotingly fixed on the housing of the pump (10, 50).

The support member (30, 70) has a u-bolt-like form. It comprises mainly an arbor (31, 71) as well as a first arm (32, 72) and a second arm (33, 73). The two arms are parallel to each other and perpendicular to the arbor. The support member (30, 70) is placed in the pump in order to be able to pivot around a first axis (A1) defined by its arbor (31, 71) from a first position, called folded-back position (figures 1, 5, 7, 9, 13, 15 and 17), to a second position, called folded-out position (figures 2, 6, 8, 10, 14, 16 and 18) and vice versa.

The frame of the display (20, 60) is fixed on the support member (30, 70) in order to be able to pivot around a second axis (A2), parallel to the first axis (A1), between a first position, called non pivoted position, and a second position, called pivoted position. By combining the rotation around the two axes (A1, A2), the frame may move from a closed position (support member in the folded-back position and frame in the non pivoted position) to an open position (support member in the folded-out position and frame in the pivoted position) via an intermediate position (support member in the folded-out position and frame in the non pivoted position).

In the first embodiment, the arbor (31) bears a bushing (34) blocked in rotation together with it. The bushing (34) may be part of the second arm (33). On its free corner on the opposite side of the second arm (33) it is provided with one depression (341) or a plurality of depressions. It cooperates with a came (15) fixed on the housing of the pump. This came (15) comprises a tubular part through which passes the arbor (31) and a part of which is projecting rearwards for fixing it on the housing of the pump. It is provided with a projection (15a) with a complementary dimension to that of the depression(s) (341). The came is blocked in rotation and cannot follow the movement of the arbor (31), but it is able to move in translation parallel to the arbor. A spring which is not represented pushes it in the direction of the bushing (34) so that its projection pushes on the corner of the bushing bearing the depression(s) (341). The penetration of the projection (105a) into one of the depressions (341) holds the support member (30) in the corresponding privileged position. The axial constraint exercised on the came (15) in order to bring it closer to the bushing (34) is sufficient for retaining the support member in one of the privileged positions so that it cannot get out of it without an exterior force being applied. It can however be surmounted manually by the user of the pump in order to make the support member pivot to another position. In the example which is shown here, the bushing (34) has only one depression corresponding to the privileged folded-back position (cf. Figure 5). It would be possible to provide a second depression corresponding to the folded-out position shown in figure 6, or even more depressions.

At the opposed end of the arbor (31), the first arm (32) of the support member is provided on its side directed towards the second arm (33) with a bolt (321) parallel to the arbor (31) and on its opposite side with a ratched wheel (322). The bolt and the ratched wheel are steady in respect to the first arm. The second arm (33) is provided on its side directed to the first arm with a hole or bearing (331) placed in the alignment of the bolt (321).

The frame (20) has on its side which is opposed to the side bearing the display (21) a first depression (22) and a second depression (23) configured to receive the free ends of the arms (32, 33) of the support member. In the second depression (23) there is a bearing (231) into which a pivot element can penetrate which is held in the bearing (331) of the second arm. The first depression has the form of an inversed L. The bolt (321) of the first arm (32) penetrates into the base of the L (not visible in the figures). This base is closed by a flange (221) fixed on the rear side of the frame forming thereby a bearing for the bolt (321). This allows the frame (20) to pivot with respect to the support member (30) around a second axis (A2) passing through the centre of the bolt (321) of the first arm and the bearing (331) of the second arm. In the non pivoted position, shown in figures 1, 5 and 7, the frame is put against the arms (32, 33) of the support member. The display (31) is essentially upright when the support member is in folded-back position. In the pivoted position shown in figures 2, 6 and 8, it has pivoted around the second axis (A2) so that it is discarded from the arms.

In order to prevent on the one hand side that the frame discards by itself from the arms and in order to maintain it on the other hand side in the desired position after a user has discarded it, a pawl system is provided. This system comprises a ratched wheel (322) fixed on the first arm (32) of the support member and a pawl (222) mounted onto the external lateral wall of the first depression (22) at the opposed side of the flange (221). A spring which is not represented maintains the pawl engaged in the ratched wheel (322). The ratched wheel (322) is oriented in such a way that the frame cannot be (more) discarded from the arm as long as the pawl is engaged. The user has to push on the lever of the pawl in order to discard the ratched wheel before being able to pivot the frame in order to discard it (more) from the arms. The tension exercised by the spring on the pawl is high enough in order to maintain the frame in the desired position, especially in a horizontal position, without making it pivot towards the arms by the effect of gravity. However, it is easily surmountable manually so that it is sufficient for a user to exercise a pressure on the frame in direction of the arms in order to push the frame against them, without the necessity to discard the pawl.

The support member (30) is placed in front of the space into which the pusher moves. In the folded-back position of the support member with the display put against the arms in the non pivoted position, as shown in figures 1, 5 and 7, the frame (20) is placed before the space reserved for the pusher so that it is practically inaccessible. In order to access to this space, it is necessary to push down the frame. In a first time, it is sufficient to push down the support member (30). In this case, the frame is directed downwards with its portion bearing the display and the keyboard, if applicable. The user who has to intervene in the space of the pusher does not see the display and is not able to access to the keyboard. He can discard the pawl (222) of the ratched wheel (322) when pushing onto the lever of the pawl, discard then the frame (20) from the arms (32, 33) of the support member until he reaches the desired incline. At this moment, the user gives free the pawl, blocking thereby the frame in the desired position. This is the position shown in figures 2, 6 and 8.

One can easily understand that the display can be discarded in a first movement from the folded-back position, shown in figures 1, 5 and 7, by making pivot the support member around the first axis (A1) and in a second movement discard it from the arms by making pivot it around the second axis (A2). These two movements are independent from each other. It is possible to make them simultaneously or subsequently. In the shown example, the housing of the pump and the form of the pusher prevent the pivot movement of the frame around the second axis (A2) as long as the support member is in the folded-back position. It is however imaginable for certain applications to allow this movement by modifying the design of the housing and of the pusher. It would then be possible to redress the frame in order to position it upwards around the second axis (A2), even if the support member is in folded-back position. This may be useful if the pump is placed in a rather low position requiring that the user gets down to it in order to read the information on the display or to enter data via the display or the keyboard.

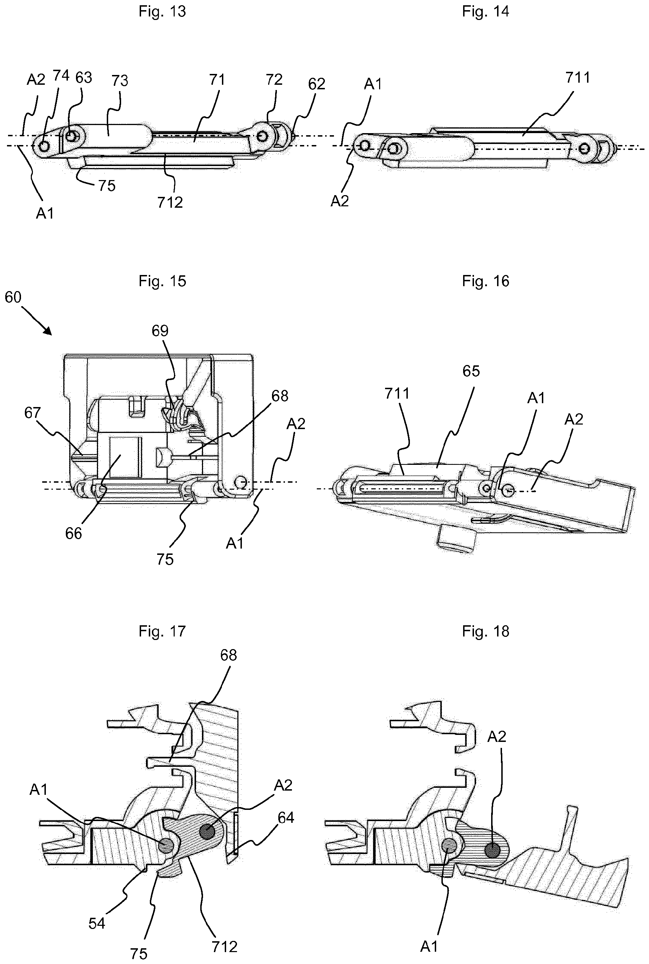

In the second embodiment, the support member (70) is fixed on the housing of the pump in order to be able to pivot around a first axis (A1) which is materialized by the arbor (71) of the support member and by a bolt (74) extending through it and fixing the support member (70) to the housing of the pump. A spring (not visible) holds the support member (70) in the folded-out position if no external force is exerted on it. As shown in figures 17 and 18, the pivoting movement of the support member (70) with respect to the housing of the pump is limited to the folded-out position by the means of a stop (54) located on the housing and another stop (75) placed on the arbor (71) of the support member. If no external force is exerted on it, the rest position of the support member is therefore the folded-out position.

The frame (60) of the display is articulated by means of two rods (62, 63) on the support member (70) in order to be able to pivot with respect to the support member (70) around a second axis (A2) parallel to the first axis (A1). The two rods (62, 63) are aligned and are parallel to the bolt (74). Irrespective of the pivoting status of the support member (70) with respect to the housing of the pump, the second pivoting axis (A2) is always placed more in front than the first pivoting axis (A1), i.e. more distant from the housing of the pump. The pivoting movement of the frame holding the display with respect to the support member is limited in both directions. In direction of the housing of the pump, the frame comes into contact with a flat part (65) against the upper side (711) of the arbor (71) so that it is in the non pivoted position. In the inverse direction, i.e. discarded from the housing of the pump, another part (64) of the frame (60), consisting in this case of the lower part of the internal side of the frame, comes into contact with the lower side (712) of the arbor (71) when the pivoted position is reached. These two positions are shown in figures 15 and 18. It is obvious that the frame can take any position between these two extreme positions.

On the side opposed to the display, the frame (70) holding the display bears a plate (66) serving as counterplate for the pumping fingers. It is provided in addition with two projections (67, 68) for blocking the tubing in the holding members (52, 53) of the pump. Furthermore, a hook (69) is provided on the frame for holding the frame in the closed position against the housing as represented in figure 9. This hook (69) acts retaining means whereas the lever provided on the side bearing the display serves as liberation means.

Like for the piston-type pump of the first embodiment, the display frame (70) is used to protect the pumping mechanism. Moreover, it must be in closed position against the housing of the pump (figure 9) in order to allow the fingers (51) to compress the tubing (55) against the counterplate (66) during the pumping procedure. However, it must be possible to discard the frame (70) in order to put in place the tubing (55) or to access to the pumping mechanism. By putting in place the tubing in the pump, it is placed in front of the fingers and maintained in this place by passing it through the two retaining members (52, 53) placed on both sides of the pumping fingers (51).

One of the fingers protrudes more than the other ones out of the pumping mechanism. At its level, the section of the tubing placed before the most protruding finger is also put more forward than the rest of the tubing. At the end of the closing movement of the display frame (70) towards the housing of the pump, the counterplate (66) comes into contact with the tubing, first at the level of this most protruding finger and then comes to press the tubing against this finger. It is important that this approach movement does not lead to a displacement of the tubing. The last may occur if the approach is oblique with respect to the horizontal.

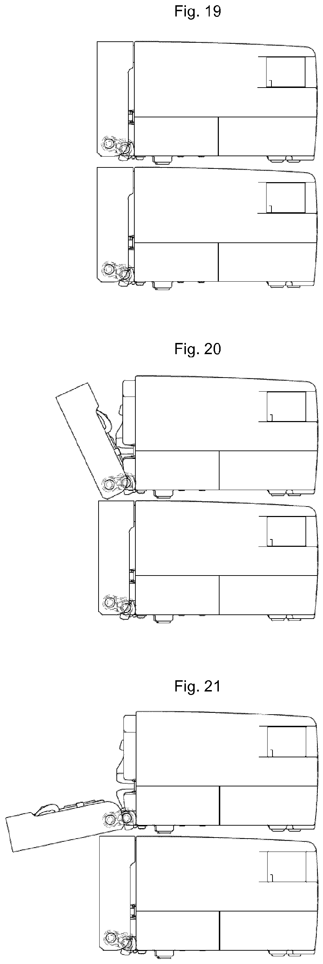

Furthermore, the display frame (70) of the finger pump is quite tight as shown in figures 9 and following. It is current to put several pumps one above the other by fixing them to masts designed therefore. If the display frame would pivot only around the first axis (A1), it would not be possible to discard sufficiently the display frame because its lower corner would come into contact with the housing of the pump directly underneath. Figure 20 represents a similar situation just before the frame hits the pump underneath. The access to the pumping mechanism would be restrained.

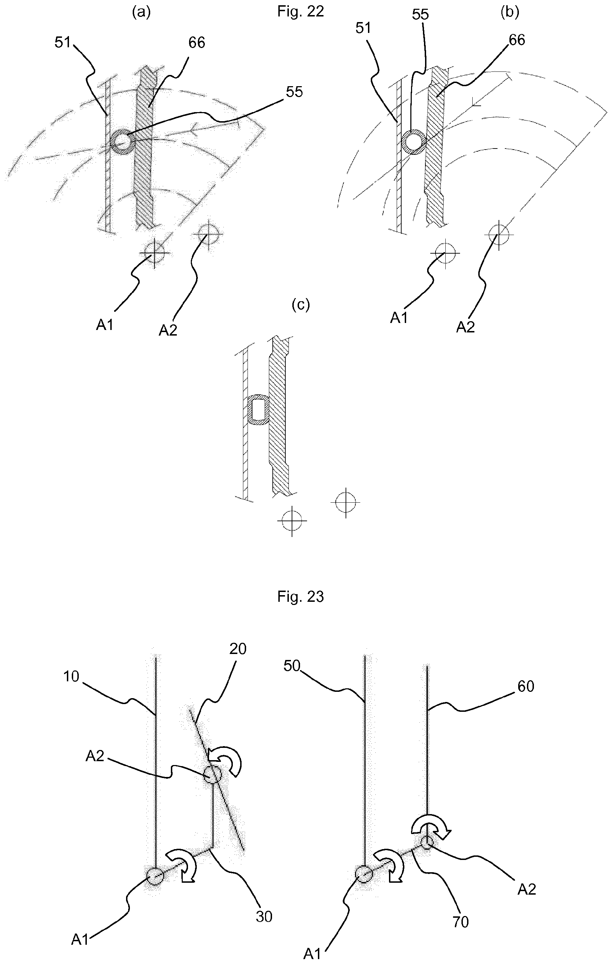

If in contrary the display frame (70) would pivot only around the second axis (A2), the projections (67, 68) and the counterplate (66) would not enter into contact with the tubing in a horizontal movement, but in an oblique movement with the risk of displacing the tubing. This situation is represented schematically in figure 22b.

The combination of the two axes (A1, A2) allows avoiding these drawbacks. When the hook (69) is liberated with the help of the lever, the spring forces the support member (70) to pass from the folded-back position to the folded-out position. The stop (75) of the support member comes into contact with the stop (54) of the housing. The pivoting movement of the support member (70) is thereby stopped. The support member (70) takes along the display frame (60). The latter is then in an intermediate position represented in figure 20 between the closed position of figures 9 and 19 and the open position of figures 10 and 21. Its lower corner has not yet hit the pump underneath, but the access to the pump mechanism is still restricted. The user can continue to discard manually the display support (60) by making it pivot around the second axis (A2) until the open position represented particularly in figures 10 and 21 is reached. This position is reached when the lower part (64) of the internal side of the display frame comes to abut against the lower side (712) of the arbor (71). For bringing back the display frame in its initial position, the user pushes the display frame upwards. Due to the effect of the spring the display pivots first around the second axis (A2) until the flat part (65) comes into contact with the upper side (711) of the arbor (71). The pivoting movement around the second axis (A2) is thereby stopped. If the user continues to push the display frame towards the housing of the pump, the pivoting movement continues around the first axis (A1), against the effect of the spring, until the display frame is fully closed and against the housing of the pump. The hook (69) snaps on so that the display frame is held in this position against the effect of the spring.

As represented in figure 22a, making the display frame (60) approach the housing of the pump by pivoting around the first axis (A1) makes sure that the press down movement of the counterplate (66) against the protruding fingers is essentially horizontal. This requires the first axis (A1) to be placed more or less vertically below the point of contact between the counterplate (66) and the part of the tubing which is adjacent to the most protruding finger.

It can be stated that in the first embodiment, the pivoting direction around the first axis (A1) and the pivoting direction around the second axis (A2) are inverted when the display passes from the closed position against the housing (figure 1) to the open position in which the support (30) is in folded-out position and the display entirely pivoted, whereas in the second embodiment these movements are in the same direction. These rotating directions are schematically represented in figures 23a and 23b.

Of course it would be possible to put the display directly on the support member (30, 70) without using a frame (20, 60).

Detecting means may be provided for detecting the position of the support member or of the display or the frame holding the display with respect to the housing of the pump. This allows particularly to stop the pump if the support member and/or the display are discarded from the folded-back position or the closed position shown in figures 1, 5 and 7or 9, 13 and 15.

**List of references:**

| | | | | | |
|---|---|---|---|---|---|
| 10 | Pump | | 50 | Pump | |
| | 11 | Bed of the syringe | | 51 | Pump fingers |
| | 12 | Axial blocking means of the syringe | | 52 | 1^{st} holding member |
| | 13 | Radial blocking means of the syringe | | 53 | 2^{nd} holding member |
| | 14 | Pusher of the pump | | 54 | Stop |
| | 15 | Indexation came | | 55 | Tubing |
| | | 15a Projection | | | |
| 20 | Frame of the display | | 60 | Display frame | |
| | 21 | Display | | 61 | Display |
| | 22 | First depression | | 62 | 1^{st} Rod |
| | | 221 Flange | | 63 | 2^{nd} Rod |
| | | 222 Pawl | | 64 | Lower part of the frame |
| | 23 | Second depression | | 65 | Flat part |
| | | 231 Bearing | | 66 | Counterplate |
| | | | | 67 | 1^{st} projection |
| | | | | 68 | 2^{nd} projection |
| | | | | 69 | Hook |
| 30 | Support member | | 70 | Support member | |
| | 31 | Arbor | | 71 | Arbor |
| | 32 | First arm | | | 711 Upper side |
| | | 321 Bolt | | | 712 Lower side |
| | | 322 Ratched wheel | | 72 | First arm |
| | 33 | Second arm | | 73 | Second arm |
| | | 331 Bearing | | 74 | Bolt |
| | 34 | Bushing | | 75 | Stop |
| | | 341 Depression | | | |
| A1 | First axis | | | | |
| A2 | Second axis | | | | |

## Claims

1. Medical pump (10, 50) comprising a pumping mechanism located in an housing and a display (21, 61) placed on a support member (30, 70) which is articulated on the housing, the support member (30, 70) being pivotable with respect to the housing around a first axis (A1) between a folded-back position and an folded-out position, **characterized in that** the display (21, 61) is pivotable with respect to the support member (30, 70) around a second axis (A2) between a first position, called non pivoted position, and a second position, called pivoted position, wherein the first axis (A1) and the second axis (A2) are parallel to each other.

2. Medical pump (10, 50) according to claim 1, **characterized in that** the display (21, 61) is placed in a frame (20, 60) which is pivotable with respect to the support member (30, 70) in order to pivot around the second axis (A2) between the non pivoted position and the pivoted position.

3. Medical pump (10) according to claim 1 or 2, **characterized in that** the support member (30) is configured to rotate around the first axis (A1) from the folded-back position to the folded-out position in a direction which is opposed to the direction of rotation of the display (21) or the frame of the display (20) from the non pivoted position to the pivoted position.

4. Medical pump (50) according to claim 1 or 2, **characterized in that** the support member (30) is configured to rotate around the first axis (A1) from the folded-back position to the folded-out position in an direction which is identical to the direction of rotation of the display (21) or the frame of the display (20) from the non pivoted position to the pivoted position.

5. Medical pump (10, 50) according to claim 2, **characterized in that** the support member (30, 70) and the display (21, 61) or the frame (20, 60) holding the display block the access to the pumping mechanism when the support member (30, 70) is in the folded-back position and the display (21, 61) or the frame (20, 60) is in the non pivoted position, whereas when the support member (30, 70) is in the folded-out position, the pumping mechanism is accessible.

6. Medical pump (10, 50) according claim 2, **characterized in that** the frame (20, 60) is provided with a keyboard which is preferably placed aside the display (21, 61).

7. Medical pump (10, 50) according to one of the preceding claims, **characterized in that** the support member (30, 70) comprises an arbor (31, 71) and two arms (32, 33, 72, 73) parallel to each other and perpendicular to the arbor (31, 71).

8. Medical pump (10) according to one of the preceding claims, **characterized in that** indexation means (34, 105) are provided for defining the privileged pivot positions of the support member (30) with respect to the housing of the pump.

9. Medical pump (10) according to claim 8 , **characterized in that** the indexation means (34, 105) comprise on the one hand side a bushing (34) fixed on the arbor (31) and being provided with at least one depression (341) and on the other hand side of a came (105) provided with a projection (105a), the came (105) being placed in the housing of the pump and being mobile exclusively in translation parallel to the axis of the arbor (31) and means being provided for pushing the projection of the came (105) against the corner of the bushing (34) provided with the depression(s) (341).

10. Medical pump (10) according to one of the preceding claims, **characterized in that** retaining means (222, 322) are provided with liberation means for preventing the display (21) or the frame (20) from pivoting in one direction with respect to the support member (30) if the liberation means are not actuated.

11. Medical pump (10) according to the preceding claim, **characterized in that** the retaining means comprise on the one hand side a ratched wheel (322) fixed on the support member and on the other hand side a pawl fixed on the display (21) or the frame (20) of the display.

12. Medical pump (10) according to the one of the preceding claims, **characterized in that** the pump is a pump of the push-syringe type and **in that** the two pivot axes (A1, A2) are parallel to the axis of a syringe placed into the pump.

13. Medical pump (50) according to one of the claims 1 to 7, **characterized in that** a spring is provided which is configured to keep the support member (70) in the folded-out position if no external force is exerted on the support member.

14. Medical pump (50) according to claim 13, **characterized in that** the pump is a peristaltic pump and **in that** the two pivot axes (A1, A2) are parallel to the camshaft of the pumping mechanism.

15. Medical pump (50) according to the preceding claim, **characterized in that** the display (61) is placed in a frame (60) which is pivotable with respect to the support member (70) in order to pivot around the second axis (A2) between the non pivoted position and the pivoted position and that a counterplate is placed on the part of the frame directed to the pumping mechanism when the support member is in folded-back position and the frame is in the non pivoted position.

16. Medical pump (50) according to the preceding claim, **characterized in that** the first axis (A1) is placed in such a way that when the display (61) or the frame (60) holding the display is in its non pivoted position and the support member (70) is moving to its folded-back position, the last part of the movement of the counterplate is essentially horizontal and perpendicular to the axis (A1).

17. Medical pump (50) according to the preceding claim, **characterized in that** the first axis (A1) is placed essentially vertically bellow the place for a tubing located against the most protruding pumping finger of the pumping mechanism.

18. Medical pump (50) according to one of the claims 13 to 17, **characterized in that** retaining means (69) are provided with liberation means for retaining the display (61) or the frame (60) holding the display in the closed position against the effect of the spring if the liberation means are not actuated.

19. Medical pump (10, 50) according to one of the preceding claims, **characterized in that** detecting means are provided for detecting the position of the support member (30, 70) or of the display with respect to the housing of the pump.

## Patentansprüche

1. Medizinische Pumpe (10, 50), umfassend einen Pumpenmechanismus, der in einem Gehäuse angeordnet ist, und einen Bildschirm (21, 61) auf einem Stützelement (30, 70), das am Gehäuse gelenkig befestigt ist, wobei das Stützelement (30, 70) bezüglich des Gehäuses um eine erste Achse (A1) zwischen einer zurückgeklappten Position und einer ausgeklappten Position schwenkbar ist, **dadurch gekennzeichnet, dass** der Bildschirm (21, 61) bezüglich des Stützelements (30, 70) um eine zweite Achse (A2) zwischen einer ersten Position, die als nicht geschwenkte Position bezeichnet wird, und einer zweiten Position, die als Schwenkposition bezeichnet wird, schwenkbar ist, wobei die erste Achse (A1) und die zweite Achse (A2) parallel zueinander sind.

2. Medizinische Pumpe (10, 50) nach Anspruch 1, **dadurch gekennzeichnet, dass** der Bildschirm (21, 61) in einem Rahmen (20, 60) platziert ist, der bezüglich des Stützelements (30, 70) schwenkbar ist, damit er um die zweite Achse (A2) zwischen der nicht geschwenkten Position und der Schwenkposition schwenken kann.

3. Medizinische Pumpe (10) nach Anspruch 1 oder 2, **dadurch gekennzeichnet, dass** das Stützelement (30) dazu ausgelegt ist, sich um die erste Achse (A1) aus der zurückgeklappten Position in die ausgeklappte Position in einer Richtung zu drehen, die der Drehrichtung des Bildschirms (21) oder des Rahmens des Bildschirms (20) aus der nicht geschwenkten Position in die Schwenkposition entgegengesetzt ist.

4. Medizinische Pumpe (50) nach Anspruch 1 oder 2, **dadurch gekennzeichnet, dass** das Stützelement (30) dazu ausgelegt ist, sich um die erste Achse (A1) aus der zurückgeklappten Position in die ausgeklappte Position in einer Richtung zu drehen, die mit der Drehrichtung des Bildschirms (21) oder des Rahmens des Bildschirms (20) aus der nicht geschwenkten Position in die Schwenkposition identisch ist.

5. Medizinische Pumpe (10, 50) nach Anspruch 2, **dadurch gekennzeichnet, dass** das Stützelement (30, 70) und der Bildschirm (21, 61) oder der den Bildschirm haltende Rahmen (20, 60) den Zugang zum Pumpenmechanismus blockieren, wenn sich das Stützelement (30, 70) in der zurückgeklappten Position befindet und sich der Bildschirm (21, 61) oder der Rahmen (20, 60) in der nicht geschwenkten Position befindet, während der Pumpenmechanismus in der ausgeklappten Position des Stützelements (30, 70) zugänglich ist.

6. Medizinische Pumpe (10, 50) nach Anspruch 2, **dadurch gekennzeichnet, dass** der Rahmen (20, 60) eine Tastatur aufweist, die vorzugsweise neben dem Bildschirm (21, 61) platziert wird.

7. Medizinische Pumpe (10, 50) nach einem der vorhergehenden Ansprüche, **dadurch gekennzeichnet, dass** das Stützelement (30, 70) einen Holm (31, 71) und zwei Arme (32, 33, 72, 73) umfasst, die parallel zueinander und senkrecht zum Holm (31, 71) sind.

8. Medizinische Pumpe (10) nach einem der vorhergehenden Ansprüche, **dadurch gekennzeichnet, dass** die Indexiermittel (34, 105) zur Definition der bevorzugten Schwenkpositionen des Stützelements (30) bezüglich des Gehäuses der Pumpe vorgesehen sind.

9. Medizinische Pumpe (10) nach Anspruch 8, **dadurch gekennzeichnet, dass** die Indexiermittel (34, 105) auf der einen Seite eine Buchse (34), die am Holm (31) befestigt ist und mit mindestens einer Vertiefung (341) ausgestattet ist, und auf der anderen Seite eine Nocke (105) umfassen, die mit einem Vorsprung (105a) ausgestattet ist, wobei die Nocke (105) im Gehäuse der Pumpe platziert ist und ausschließlich verschiebbar parallel zur Achse des Holms (31) beweglich ist, und wobei Mittel zum Schieben des Vorsprungs der Nocke (105) gegen die Ecke der Buchse (34), die mit der (den) Vertiefung(en) (341) ausgestattet ist, vorgesehen sind.

10. Medizinische Pumpe (10) nach einem der vorhergehenden Ansprüche, **dadurch gekennzeichnet, dass** Haltemittel (222, 322) mit Freigabemitteln zur Verhinderung einer Schwenkung des Bildschirms (21) oder des Rahmens (20) in eine Richtung bezüglich des Stützelements (30), wenn die Freigabemittel nicht betätigt werden, vorgesehen sind.

11. Medizinische Pumpe (10) nach einem der vorhergehenden Ansprüche, **dadurch gekennzeichnet, dass** die Haltemittel auf einer Seite ein Sperrrad (322), das auf dem Stützelement befestigt ist, und auf der anderen Seite eine Sperrklinke umfassen, die am Bildschirm (21) oder am Rahmen (20) des Bildschirms befestigt ist.

12. Medizinische Pumpe (10) nach einem der vorhergehenden Ansprüche, **dadurch gekennzeichnet, dass** die Pumpe eine Pumpe vom Druckspritzentyp ist und dass die zwei Drehachsen (A1, A2) parallel zu der Achse einer in der Pumpe platzierten Spritze sind.

13. Medizinische Pumpe (50) nach einem der Ansprüche 1 bis 7, **dadurch gekennzeichnet, dass** eine Feder vorgesehen ist, die dazu ausgelegt ist, das Stützelement (70) in der ausgeklappten Position zu halten, wenn keine externe Kraft auf das Stützelement aufgebracht wird.

14. Medizinische Pumpe (50) nach Anspruch 13, **dadurch gekennzeichnet, dass** die Pumpe eine Peristaltikpumpe ist und dass die zwei Schwenkachsen (A1, A2) parallel zu der Nockenwelle des Pumpmechanismus sind.

15. Medizinische Pumpe (50) nach dem vorhergehenden Anspruch, **dadurch gekennzeichnet, dass** der Bildschirm (61) in einem Rahmen (60) platziert ist, der bezüglich des Stützelements (70) schwenkbar ist, damit er um die zweite Achse (A2) zwischen der nicht geschwenkten Position und der Schwenkposition schwenkt, und dass eine Gegenplatte auf dem Teil des Rahmens angeordnet ist, der zum Pumpenmechanismus gerichtet ist, wenn sich das Stützelement in der zurückgeklappten Position befindet und sich der Rahmen in der nicht geschwenkten Position befindet.

16. Medizinische Pumpe (50) nach dem vorhergehenden Anspruch, **dadurch gekennzeichnet, dass** die erste Achse (A1) derart angeordnet ist, dass, wenn sich der Bildschirm (61) oder der den Bildschirm haltende Rahmen (60) in seiner nicht geschwenkten Position befindet und sich das Stützelement (70) in seine zurückgeklappte Position bewegt, der letzte Teil der Bewegung der Gegenplatte im Wesentlichen horizontal und senkrecht zur Achse (A1) ist.

17. Medizinische Pumpe (50) nach dem vorhergehenden Anspruch, **dadurch gekennzeichnet, dass** die erste Achse (A1) im Wesentlichen vertikal unter der Stelle für einen Schlauch gegen den am meisten vorragenden Pumpenfinger des Pumpmechanismus angeordnet ist.

18. Medizinische Pumpe (50) nach einem der Ansprüche 13 bis 17, **dadurch gekennzeichnet, dass** Haltemittel (69) mit Freigabemitteln zum Festhalten des Bildschirms (61) oder des den Bildschirm haltenden Rahmens (60) in der geschlossenen Position gegen die Wirkung der Feder, wenn die Freigabemitteln nicht betätigt werden, vorgesehen sind.

19. Medizinische Pumpe (50) nach einem der vorhergehenden Ansprüche, **dadurch gekennzeichnet, dass** Detektionsmittel zum Erkennen der Position des Stützelements (30, 70) oder des Bildschirms bezüglich des Gehäuses der Pumpe vorgesehen sind.

## Revendications

1. Pompe médicale (10, 50) comprenant un mécanisme de pompage situé dans un boîtier et un écran d'affichage (21, 61) placé sur un élément de support (30, 70) qui est articulé sur le boîtier, l'élément de support (30, 70) pouvant pivoter par rapport au boîtier autour d'un premier axe (A1) entre une position repliée et une position dépliée, **caractérisée en ce que** l'écran d'affichage (21, 61) peut pivoter par rapport à l'élément de support (30, 70) autour d'un second axe (A2) entre une première position, appelée position non pivotée, et une seconde position, appelée position pivotée, dans laquelle le premier axe (A1) et le second axe (A2) sont parallèles l'un à l'autre.

2. Pompe médicale (10, 50) selon la revendication 1, **caractérisée en ce que** l'écran d'affichage (21, 61) est placé dans un cadre (20, 60) qui peut pivoter par rapport à l'élément de support (30, 70) dans le but de pivoter autour du second axe (A2) entre la position non pivotée et la position pivotée.

3. Pompe médicale (10) selon la revendication 1 ou 2, **caractérisée en ce que** l'élément de support (30) est configuré de manière à tourner autour du premier axe (A1) à partir de la position repliée jusqu'à la position dépliée dans une direction qui est opposée au sens de rotation de l'écran d'affichage (21) ou du cadre de l'écran d'affichage (20) à partir de la position non pivotée jusqu'à la position pivotée.

4. Pompe médicale (50) selon la revendication 1 ou 2, **caractérisée en ce que** l'élément de support (30) est configuré de manière à tourner autour du premier axe (A1) à partir de la position repliée jusqu'à la position dépliée dans une direction qui est identique au sens de rotation de l'écran d'affichage (21) ou du cadre de l'écran d'affichage (20) à partir de la position non pivotée jusqu'à la position pivotée.

5. Pompe médicale (10, 50) selon la revendication 2, **caractérisée en ce que** l'élément de support (30, 70) et l'écran d'affichage (21, 61) ou le cadre (20, 60) qui maintient l'écran d'affichage bloquent l'accès au mécanisme de pompage lorsque l'élément de support (30, 70) se trouve dans la position repliée et que l'écran d'affichage (21, 61) ou le cadre (20, 60) se trouve dans la position non pivotée, tandis que lorsque l'élément de support (30, 70) se trouve dans la position dépliée, le mécanisme de pompage est accessible.

6. Pompe médicale (10, 50) selon la revendication 2, **caractérisée en ce que** le cadre (20, 60) est pourvu d'un clavier qui est de préférence placé à côté de l'écran d'affichage (21, 61).

7. Pompe médicale (10, 50) selon l'une des revendications précédentes, **caractérisée en ce que** l'élément de support (30, 70) comprend un arbre (31, 71) et deux bras (32, 33, 72, 73) parallèles l'un à l'autre et perpendiculaires à l'arbre (31, 71).

8. Pompe médicale (10) selon l'une des revendications précédentes, **caractérisée en ce que** des moyens d'indexation (34, 105) sont prévus pour définir les positions de pivotement privilégiées de l'élément de support (30) par rapport au boîtier de la pompe.

9. Pompe médicale (10) selon la revendication 8, **caractérisée en ce que** les moyens d'indexation (34, 105) comprennent d'une part une douille (34) fixée sur l'arbre (31) et pourvue d'au moins une dépression (341) et d'autre part d'une came (105) pourvue d'une saillie (105a), la came (105) étant placée dans le boîtier de la pompe et étant mobile exclusivement par des déplacements parallèles à l'axe de l'arbre (31), et des moyens sont prévus pour pousser la saillie de la came (105) contre le coin de la douille (34) pourvue de la ou des dépression(s) (341).

10. Pompe médicale (10) selon l'une des revendications précédentes, **caractérisée en ce que** des moyens de retenue (222, 322) sont prévus avec des moyens de libération pour empêcher l'écran d'affichage (21) ou le cadre (20) de pivoter dans une direction par rapport à l'élément de support (30) si les moyens de libération ne sont pas actionnés.

11. Pompe médicale (10) selon la revendication précédente, **caractérisée en ce que** les moyens de retenue comprennent d'une part une roue à rochet (322) fixée sur l'élément de support et d'autre part un cliquet fixé sur l'écran d'affichage (21) ou sur le cadre (20) de l'écran d'affichage.

12. Pompe médicale (10) selon l'une des revendications précédentes, **caractérisée en ce que** la pompe est une pompe du type pousse-seringue, et **en ce que** les deux axes de pivotement (A1, A2) sont parallèles à l'axe d'une seringue placée dans la pompe.

13. Pompe médicale (50) selon l'une des revendications 1 à 7, **caractérisée en ce qu'**un ressort est prévu et est configuré de manière à maintenir l'élément de support (70) dans la position dépliée si aucune force externe n'est exercée sur l'élément de support.

14. Pompe médicale (50) selon la revendication 13, **caractérisée en ce que** la pompe est une pompe péristaltique, et **en ce que** les deux axes de pivotement (A1, A2) sont parallèles à l'arbre à cames du mécanisme de pompage.

15. Pompe médicale (50) selon la revendication précédente, **caractérisée en ce que** l'écran d'affichage (61) est placé dans un cadre (60) qui peut pivoter par rapport à l'élément de support (70) dans le but de pivoter autour du second axe (A2) entre la position non pivotée et la position pivotée, et **en ce qu'**une contreplaque est placée sur la partie du cadre dirigée vers le mécanisme de pompage lorsque l'élément de support se trouve dans la position repliée et que le cadre se trouve dans la position non pivotée.

16. Pompe médicale (50) selon la revendication précédente, **caractérisée en ce que** le premier axe (A1) est placé de telle sorte que lorsque l'écran d'affichage (61) ou le cadre (60) qui maintient l'écran d'affichage se trouve dans sa position non pivotée et que l'élément de support (70) se déplace vers sa position repliée, la dernière partie du déplacement de la contreplaque est essentiellement horizontale et perpendiculaire à l'axe (A1).

17. Pompe médicale (50) selon la revendication précédente, **caractérisée en ce que** le premier axe (A1) est placé essentiellement verticalement en dessous de l'emplacement d'un tubage situé contre le doigt de pompage le plus saillant du mécanisme de pompage.

18. Pompe médicale (50) selon l'une des revendications 13 à 17, **caractérisée en ce que** des moyens de retenue (69) sont pourvus de moyens de libération pour retenir l'écran d'affichage (61) ou le cadre (60) qui maintient l'écran d'affichage dans la position fermée contre l'effet du ressort si les moyens de libération ne sont pas actionnés.

19. Pompe médicale (10, 50) selon l'une des revendications précédentes, **caractérisée en ce que** des moyens de détection sont prévus pour détecter la position de l'élément de support (30, 70) ou de l'écran d'affichage par rapport au boîtier de la pompe.
